# EUROPEAN PATENT SPECIFICATION

(11) **EP 1 516 626 B1**
(45) Date of publication and mention of the grant of the patent: **27.12.2006**
(21) Application number: 03021000.9
(22) Date of filing: 17.09.2003
(51) Int. Cl.: A61K 38/14, A61P 31/04

(54) **A Teicoplanin composition with improved antibiotic activity**
Teicoplanin Zubereitung mit verbesserter antibiotischer Wirkung
Préparation contenant la Teicoplanin possèdant une activité antibiotique ameliorée

(43) Date of publication of application: 23.03.2005
(73) Proprietor: Alpharma APS, 2300 Copenhagen S (DK)
(72) Inventor: Suhr-Jessen, Trine, 2820 Gentofte (DK); Koch, Torben, 2400 Copenhagen NV (DK); Thyme, Jorn, 4000 Roskilde (DK); Mathiesen, Anita, 1369 Stabekk (NO); Reijns, Tiemen Geen Pieter, 2544 GM Den Haag (NL)
(74) Representative: Pitman, Richard Stephen

(56) References cited:
- EP-A- 0 316 712
- US-A- 4 239 751
- US-A- 4 698 418
- CORONELLI, C. ET AL: "Teicoplanin: chemical, physicochemical and biological aspects" FARMACO, EDIZIONE SCIENTIFICA (1987), 42(10), 767-86 , XP009025779
- MALABARBA A ET AL: "Teicoplanin, antibiotics from Actinoplanes teichomyceticus nov. sp. VI. Chemical degradation: Physico-chemical and biological properties of acid hydrolysis products" JOURNAL OF ANTIBIOTICS 1984 JAPAN, vol. 37, no. 9, 1984, pages 988-999, XP009025805

## Description

### FIELD OF THE INVENTION:

The present invention relates to a Teicoplanin composition with improved antibiotic activity, use thereof as a pharmaceutical, and a method for producing a Teicoplanin composition with improved antibiotic activity.

### BACKGROUND OF THE INVENTION:

Teicoplanin is a glycopeptide antibiotic produced by *Actinoplanes teicomyceticus* and was discovered during a scientific research program aiming to find new molecules of microbial origin that inhibited bacterial cell wall synthesis (Reference 1). It was first described in 1978 and ten years later it was introduced into clinical practice in Italy (Reference 2). According to Lancini (Reference 3) glycopeptide antibiotics can be classified in four groups based on their chemical structure:
group I (or the vancomycin type) has aliphatic amino acids at positions 1 and 3;
group II (or the avoparcin type) has aromatic amino acid residues at positions 1 and 3;
group III (or the ristocetin type) is similar to group II but for an ether linkage joining the aromatic amino acids at positions 1 and 3
group IV (or the Teicoplanin type) has the same amino acid arrangement as group III plus a
fatty acid residue attached to the amino sugar.
Teicoplanin shares many chemical and microbiological characteristics with vancomycin but it possesses higher activity against many Gram-positive bacteria and is less nephrotoxic (Reference 2 and Reference 4).

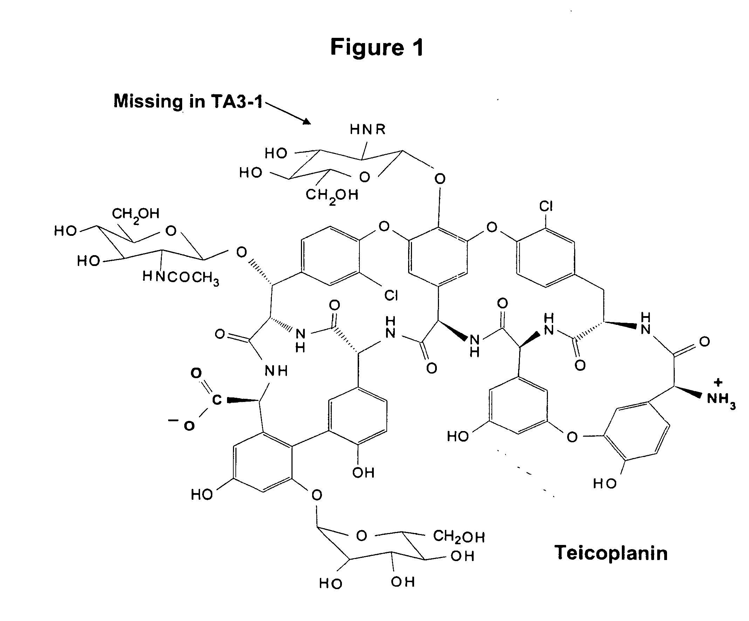

The molecular structure of Teicoplanin is depicted in Figure 1 herein. Like all glycopeptide antibiotics Teicoplanin contains a core linear heptapeptide, in which five of the seven amino acids are common to all the members of its group. The remaining two (positions 1 and 3) are linked together by an ether bond (Reference 3). Three sugar moieties are attached to the aryl groups of amino acids 7, 6 and 4, namely a D-mannose, an N-acetyl-D-glucosamine and an N-acyl-D-glucosamine respectively. These sugars have no biological activity in vitro, but are important in imparting different pharmacokinetic properties (Reference 5).

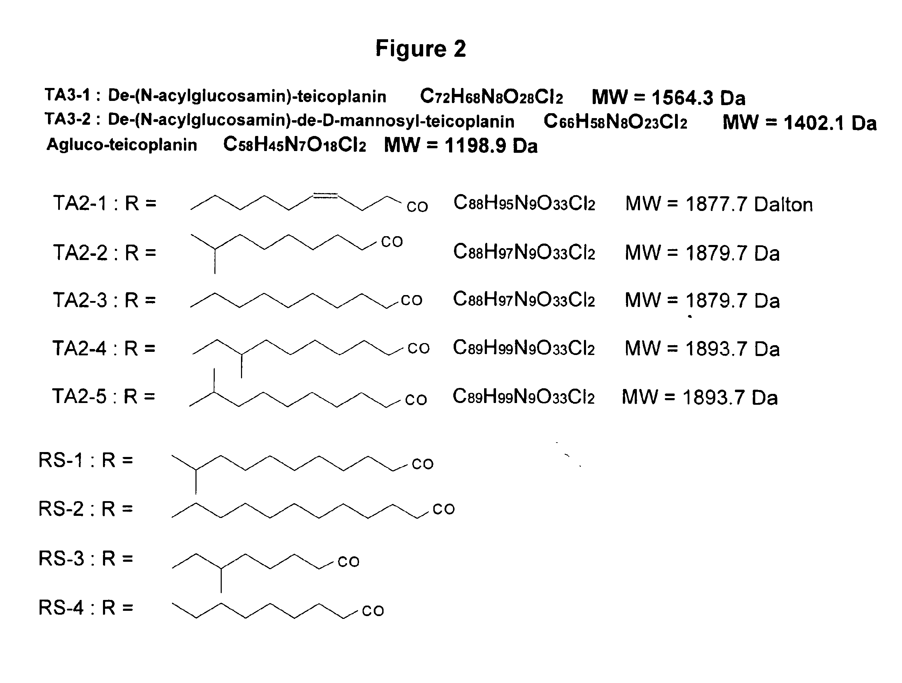

Teicoplanin comprises different components, including five closely related molecules denoted Teicoplanin A2-1 (TA2-1) to Teicoplanin A2-5 (TA2-5), which differ only in the nature of the acyl moiety (C₁₀ and C₁₁fatty acids); RS-1 to RS-4; a more polar component Teicoplanin A3-1 (TA3-1), which lacks the *N*-acyl-D-glucosamine residue and Teicoplanin TA3-2, which also lacks the D-mannose group. Substitution of the sugar residues with hydrogens results in the core aglycone, which is considered as a Teicoplanin A3 component. The molecular structures of the different components are shown in Figure 2 herein.

Reference 7 discloses that Teicoplanin is an antibiotic complex consisting of five closely related factors which may be transformed into pseudoaglycones and finally into a single aglycone with consecutive removal of three sugar units by acid hydrolysis.

US4239751 (Reference 9) describes isolation of different Teicoplanin components. Table IV shows that TA3 has lower antibiotic activity than TA2.

US4994555 (Reference 10) reads on column 2, lines 51-53: "As employed in its biological application Teicoplanin essentially consists of factor A2 (TA2) accompanied by minor amount of factor A3 (T-A3)".

Reference 11 discloses structure determination and the physico-chemical properties of Teicoplanin.

Targocid® is a commercial Teicoplanin product from Aventis Pharma.

### SUMMARY OF THE INVENTION:

The problem to be solved by the present invention is to provide a Teicoplanin composition with improved antibiotic activity.

The solution is based on that the present inventors have identified that a positive synergistic effect can be obtained by making a Teicoplanin composition with a proper ratio of the amounts of TA2 and TA3. Experimental work of the present inventors confirmed the prior art teaching that relatively pure TA3 as such has lower antibiotic activity than TA2 as such. Despite of this, the present inventors made different Teicoplanin compositions, where they added increasing amounts of TA3 to get an increased TA3:TA2 ratio. Contrary to the teaching of the art, they found that within a certain window of %(mg TA3)/(mg TA3 + TA2), it was possible to make a Teicoplanin composition with improved antibiotic activity due to a synergistic effect of TA2 and TA3 in the composition.

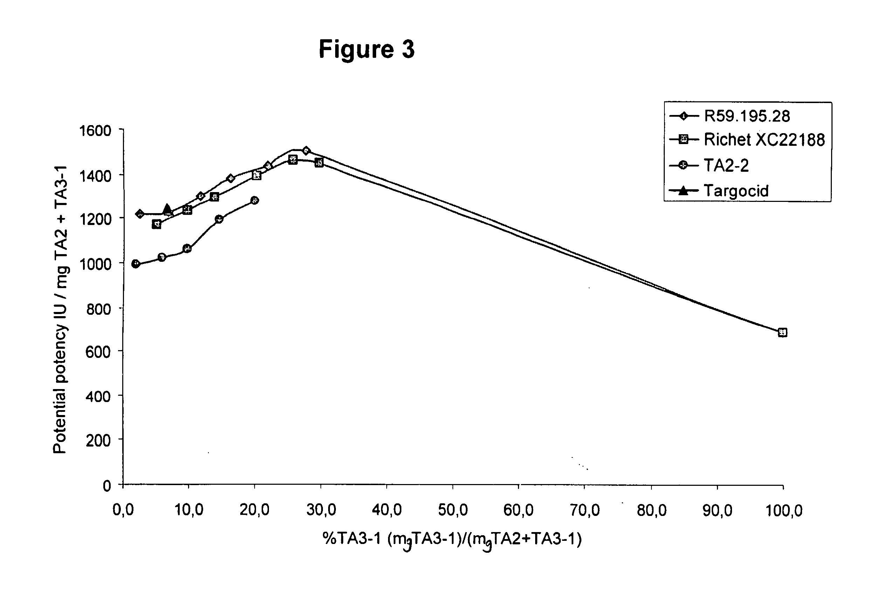

Figure 3 is a graph giving an illustrative example of this. The commercial Teicoplanin product Targocid® was used as a reference composition. Based on a standard in the art known HPLC analysis of Targocid® it was determined that Targocid® has a %(mg TA3-1)/(mg TA3-1 + TA2) of around 6.5%. The data of figure 3 shows that by mixing in TA3-1 it was possible to make a Teicoplanin composition with higher antibiotic activity than Targocid®. More specifically, within the range of %(mg TA3)/(mg TA3 + TA2) from 6.75% to 60% the tested compositions had a clearly higher antibiotic activity than Targocid®.

In order to purify TA2 and TA3 components the present inventors analyzed (by HPLC) a commercial Teicoplanin composition from Xingchang Pharma (China). This composition had a %(mg TA3)/(mg TA3 + TA2) of around 10%.

The presents inventors are not aware of any other public known commercial relevant Teicoplanin pharmaceutical compositions having a %(mg TA3)/(mg TA3 + TA2) of higher than 10%.

Accordingly, a first aspect of the invention relates to a Teicoplanin composition, comprising an effective amount of Teicoplanin components or a pharmaceutically acceptable salt or derivative thereof and a pharmaceutically acceptable carrier, wherein the composition comprises %(mg TA3)/(mg TA3 + TA2) in the range from 11 % to 60%.

A second aspect of the invention relates to use of a Teicoplanin pharmaceutical composition of the first aspect of the invention for the manufacture of a medicament for the treatment of a microorganism infection in a patient.

As explained above, the present inventors have identified that it is possible to make a Teicoplanin composition where there is a positive synergistic effect of TA2 and TA3. This teaching may be used to make a Teicoplanin composition with improved antibiotic activity.

Accordingly, a third aspect of the invention relates to a method for making a Teicoplanin composition with improved antibiotic activity comprising following steps:
(i) making two or more Teicoplanin composition(s) comprising different %(mg TA3)/(mg TA3 + TA2) in the range from 5% to 60%, measured by HPLC analysis;
(ii) analyzing the antibiotic activity of the compositions;
(iii) identifying a composition where there is a synergistic effect of TA2 and TA3 and which have a desired improved activity.

A fourth aspect of the invention relates to a method for producing a Teicoplanin composition comprising TA2 and TA3, said method comprises hydrolysis of a composition containing TA2, e.g. by treatment with an acid or an enzyme.

### DRAWINGS:

- Figure 1:: Molecular structure of the Teicoplanin complex.
- Figure 2:: Molecular structure of the different Teicoplanin components.
- Figure 3:: Graphical presentation of data that shows that by mixing in TA3-1 it was possible to make a Teicoplanin composition with higher antibiotic activity than Targocid®. For further details see section 3 of example 1 herein.
- Figure 4:: Shows HPLC diagram of Targocid®, a Teicoplanin composition (Richet) and a Teicoplanin composition described in further details in working examples herein.

### DETAILED DESCRIPTION OF THE INVENTION:

### Teicoplanin composition:

The term TA2 is a standard short name for Teicoplanin A2. Herein, this term comprises Teicoplanin having three carbohydrate moieties, such as the five closely related molecules denoted Teicoplanin A2-1 (TA2-1) to Teicoplanin A2-5 (TA2-5) and RS-1 to RS-4. Also, the term TA2 comprises derivatives thereof, such as derivatives that have been altered in the substituent R (see fig. 1). Examples on R are given in the prior art and are known to the skilled person. Examples on molecular structures of different TA2 components are shown in Figure 1 and 2 herein. Preferably the term TA2 denotes herein the Teicoplanin components selected from the group consisting of Teicoplanin A2-1 (TA2-1) to Teicoplanin A2-5 (TA2-5) and RS-1 to RS-4, or the term denotes the sum of two or more Teicoplanins components with three carbohydrate moities, such as the sum of the 2, 3, 4, 5, 6, 7 or more of the most predominant Teicoplanins in the composition. More preferably the term TA2 denotes herein the Teicoplanin components selected from the group consisting of Teicoplanin A2-1 (TA2-1) to Teicoplanin A2-5 (TA2-5), or the sum of these, i.e. the Teicoplanin A2 group (A2-1 + A2-2 + A2-3 + A2-4 + A2-5). Even more preferably the term TA2 denotes herein only the Teicoplanin component TA2-2.

The term TA3 is a standard short name for Teicoplanin A3. Herein, this term comprises Teicoplanin components having two (e.g. TA3-1), one (e.g. TA3-2) or no carbohydrate moieties (e.g. Teicoplanin aglycone), either individually or in combination (e.g. the amount of all Teicoplanins having two or less carbohydrate moieties or the sum of TA3-1 and TA3-2). Preferably the term TA3 denotes herein only the Teicoplanin component TA3-1. The molecular structures of TA3-1, which is the presently preferred TA3 component, and TA3-2 are shown in Figure 1 and 2 herein.

The specific amounts of TA3 and TA2 in the composition can be determined by any suitable means, presently HPLC analysis is preferred. Briefly, such an HPLC analysis comprises following steps: (i) having a suitable sample solution of a specified amount of the composition, (ii) performing the HPLC test with a suitable amount of the sample solution according suitable standard operating conditions, and measure the peak area of TA2 and the peak area of TA3 by a suitable automatic integration method.

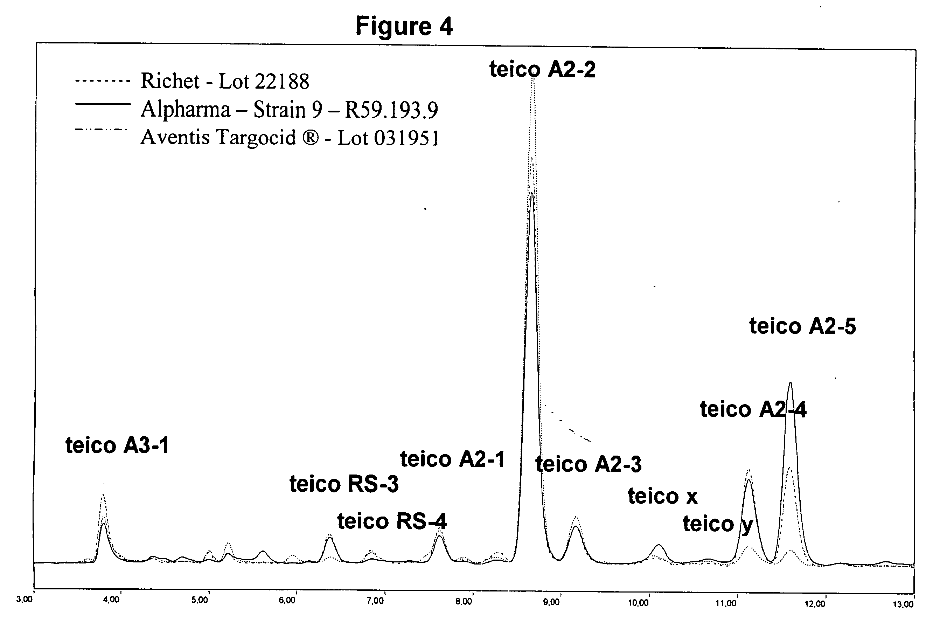

A suitable preferred HPLC-analysis protocol is provided in section 1.3.3 of example 1 herein. A typical HPLC diagram is shown in Figure 4. Figure 4 shows HPLC diagram of Targocid®, a Teicoplanin composition (Richet) and one Teicoplanin composition described in further details in working examples herein.

In a preferred embodiment, the Teicoplanin composition comprises %(mg TA3)/(mg TA3 + TA2) in the range from 13 % to 50%, measured by HPLC analysis, more preferably the Teicoplanin composition comprises %(mg TA3)/(mg TA3 + TA2) in the range from 15 % to 40%, measured by HPLC analysis, even more preferably the Teicoplanin composition comprises %(mg TA3)/(mg TA3 + TA2) in the range from 18 % to 40% and most preferably the Teicoplanin composition comprises %(mg TA3)/(mg TA3 + TA2) in the range from 20 % to 40%. Other examples on ranges have the lower limit selected from the group consisting of the values: 12%; 14%; 16%; 17%; 19%; 21%; 22%; 23%; 24% or 25%, and the upper limit selected from the group consisting of the values: 35%; 45%; 50%; 55%; or 30%, these values can be combined freely to form ranges.

Preferably the Teicoplanin composition comprises more than 600 mg dry matter of active TA2 and TA3 components per g dry matter composition.

It may be preferred to make the Teicoplanin composition in a relatively large amount in order to e.g. provide the Teicoplanin composition as a so-called bulk product. Such a bulk product can be used to make e.g. several individual pharmaceutical Teicoplanin compositions. Accordingly it may be preferred that the Teicoplanin composition has a total weight of the composition of at least 100g, more preferably of at 500g. The individual pharmaceutical composition preferably has a content of 1mg to 1000 mg of Teicoplanin.

As explained above the commercial Teicoplanin pharmaceutical composition Targocid® has been used as reference in experimental examples described herein and based on the TA2 and TA3 identified synergistic effects, the present inventors were routinely able to prepare compositions with a better antibiotic activity than Targocid®. Measured by HPLC-analysis protocol of section 1.3.3 of example 1 herein, Targocid® has a %(mg TA3-1)/(mg TA3-1 + TA2) of 6.5%.

Accordingly, a preferred embodiment of the invention relates to a Teicoplanin composition as described herein, wherein the composition has a better antibiotic activity than a corresponding Teicoplanin composition that has a %(mg TA3)/(mg TA3 + TA2) of 6.5%, where the antibiotic activity is measured by a microbiological agar diffusion assay using *Staphylococcus aureus* ATCC 6538 as test strain and the measured potential potency (IU per mg TA2 + TA3/g composition) is used to calculate the antibiotic activity.

A suitable microbiological agar diffusion assay is provided in section 1.3.4.1 of example 1 herein.

The logic behind the potential potency (IU per mg TA2 + TA3) unit may be illustrated by looking at Table 2 of section 2 of example 1 herein. Here it can be seen that the antibiotic potency is measured for the composition as such (IU/mg composition). Since the amounts of TA2 and TA3 of the composition also is known it is possible to calculate the potential potency (IU per mg TA2 + TA3) as done in this Table 2.

Preferably, the potential potency of the Teicoplanin composition, as described herein, is at least 5 IU higher than the corresponding Teicoplanin composition that has a %(mg TA3)/(mg TA3 + TA2) of 6.5%, more preferably it is at least 30 IU higher than the corresponding Teicoplanin composition that has a %(mg TA3)/(mg TA3 + TA2) of 6.5%, even more preferably it is at least 50 IU higher than the corresponding Teicoplanin composition that has a %(mg TA3)/(mg TA3 + TA2) of 6.5% and most preferably it is at least 100 IU higher than the corresponding Teicoplanin composition that has a %(mg TA3)/(mg TA3 + TA2) of 6.5%. Under some circumstances it may be difficult to get more than 1000 IU higher activity.

The term "corresponding Teicoplanin composition that has a %(mg TA3)/(mg TA3 + TA2) of 6.5%," should be understood as a standard reference composition in the sense that, beside the specific TA3 + TA2 amounts, it should comprise the same components, salt and etc. as the Teicoplanin composition which is evaluated in relation to its a better antibiotic activity.

The Teicoplanin components may be converted into salts, such as pharmaceutically acceptable salts and derivatives, like esters and ethers, and other chemical equivalents, which, within a Teicoplanin composition as described herein, are all covered by the present invention. The salts and derivatives can be prepared by standard procedures known to one skilled in the art. Salts like sodium and potassium salts, for example, may be prepared by treating Teicoplanin components with suitable sodium or potassium bases.

Esters and ethers may be prepared by the methods given in the literature, for example, in Advanced Organic Synthesis, 4^{th} Edition, J. March, John Wiley & Sons., 1992.
The amino group of the sugar moiety can be alkylated or acetylated, e.g. with acid chlorides by standard procedures known to one skilled in the art.
Chemical equivalents may be stable complexes with metal ions, e.g. transition metals like La³+, Sm³+, Eu³+, Gd ³+, which are typical for tetramic acid derivatives and may be prepared by the methods given in the literature (K. Tanaka et. al., Chem. Pharm. Bull. 1979, 27,1901; K. Matsuo, Chem. Pharm. Bull. 1980, 28, 2494).
The double bonds of the alkyl side chain may be reduced by the methods given in the literature, for example in P. N. Rylander, "Hydrogenation Methods", Academic Press, New York (1985), Chpt. 2, or may be hydrohalogenated by methods described by H. O. House in "Modern Synthetic Reactions", W. A. Benjymin, Inc., New York (1972), pp 446-452. Hydroxylated derivatives may be produced by reaction of the double bonds with reagents such as OsO₄ as described in the literature, e.g. in Chem. Rev. 1980, 80, 187.
Derivatives may also be formed by conversion of the double bonds into epoxides by oxidation, e.g. with MCPBA, as described in Advanced Organic Synthesis, 4^{th} Edition, J. March, John Wiley & Sons., 1992.

The Teicoplanin composition may be a Teicoplanin pharmaceutical composition. In e.g. such a case the Teicoplanin composition may optionally comprises pharmaceutically acceptable carriers. By "pharmaceutically acceptable carrier" as used herein is meant one or more compatible solid or liquid filler diluents, or encapsulating substances. By "compatible" as used herein is meant that the components of the composition are capable of being commingled without interacting in a manner which would substantially decrease the pharmaceutical efficacy of the total composition under ordinary use situations.

Some examples of substances which can serve as pharmaceutical carriers are salts; sugars, such as lactose, glucose and sucrose; starches such as corn starch and potato starch; cellulose and its derivatives such as sodium carboxymethycellulose, ethylcellulose and cellulose acetates; powdered tragancanth; malt; gelatin; talc; stearic acids; magnesium stearate; calcium sulfate; vegetable oils, such as peanut oils, cotton seed oil, sesame oil, olive oil, corn oil and oil of theobroma; polyols such as propylene glycol, glycerine, sorbitol, manitol, and polyethylene glycol; agar; alginic acids; water; pyrogen-free water; isotonic saline; and phosphate buffer solution; skim milk powder; as well as other non-toxic compatible substances used in pharmaceutical formulations.

Wetting agents, fillers and lubricants such as sodium lauryl sulfate, as well as coloring agents, flavoring agents, lubricants, excipients, tabletting agents, stabilizers, anti-oxidants and preservatives, can also be present.

Teicoplanin pharmaceutical composition as described herein can be administered intramuscularly, intravenously, orally, or by other modes of administration.

A Teicoplanin pharmaceutical composition, as described herein, with other pharmaceutically active substances can be prepared by mixing the active compounds with one or more pharmacologically tolerated auxiliaries and/or excipients such as, for example, fillers, emulsifiers, lubricants, masking flavors, colorants, or buffer substances, and converting the mixture into a suitable pharmaceutical form such as, for example, tablets, coated tablets, capsules, granules, powders, emulsions, suspensions, or solutions suitable for parenteral administration.

Examples of auxiliaries and/or excipients which may be mentioned are tragacanth, lactose, talc, agar, polyglycols, ethanol, and water. Suitable and preferred for parenteral administration are suspensions or solutions in water. It is also possible to administer the active substances as such, without vehicles or diluents, in a suitable form, for example, in capsules.

Preferably, the Teicoplanin composition is in the form of a powder (such as a powder for injection), in particular a powder comprising sodium chloride or glucose. Presently preferred is powders with Sodium chloride (0.5% to 15%).

Preferably, the Teicoplanin pharmaceutical composition, as described herein, is provided together with suitable pharmaceutically relevant instructions. The instructions preferably explain pharmaceutically relevant information such as e.g. qualitative and quantitative of composition, pharmaceutical form, therapeutic indications and method of administration (including recommend doses).

### Use of a Teicoplanin pharmaceutical composition for the treatment of a microorganism infection in a patient.

A Teicoplanin pharmaceutical composition, as described herein, can be administered to animals, such as mammals, including humans, as pharmaceuticals on their own, in mixtures with one another, and in the form of pharmaceutical compositions that permit parenteral administration.

Accordingly, the term "patient" relates to an animal, such as a mammal, including a human patient. Preferably it is a human patient.

Teicoplanin is known to be particular effective against an infection with Gram-positive bacteria, in particular with staphylococcal bacteria.

A Teicoplanin pharmaceutical composition can be administered orally, intramuscularly, intravenously, or by other modes of administration.

As is customary, the galenic formulation and the method of administration as well as the dosage range which are suitable in a specific case depend on the species to be treated and on the state of the respective condition or disease, and can be optimized using methods known in the art.

The UK electronic Medicines Compendium say it relation to Targocid® that a suitable recommendable daily dose may be around 3mg Targocid® / kg weight of a normal adult.

Accordingly, on average, the daily dose of Teicoplanin pharmaceutical composition in a patient may be from 0.05 mg / kg weight to 50 mg / kg weight, more preferably from 1 mg / kg weight to 20 mg / kg.

### A method for making a Teicoplanin composition with improved antibiotic activity:

As said above a third aspect of the invention relates to a method for making a Teicoplanin composition with improved antibiotic activity comprising following steps:
(i) making two or more Teicoplanin compositions comprising different %(mg TA3)/(mg TA3 + TA2) in the range from 5 % to 60%, measured by HPLC analysis;
(ii) analyzing the antibiotic activity of the compositions;
(iii) identifying a composition where there is a synergistic effect of TA2 and TA3 and which have a desired improved antibiotic activity.

The global idea behind this method relates to the herein-identified synergistic effect of TA2 and TA3.

Preferably in step (i) is made two or more Teicoplanin composition(s) comprising different %(mg TA3)/(mg TA3 + TA2) in the range from 7.5 % to 50%, more preferably in step (i) is made two or more Teicoplanin composition(s) comprising different %(mg TA3)/(mg TA3 + TA2) in the range from 15 % to 40% and even more preferably in step (i) is made two or more Teicoplanin composition(s) comprising different %(mg TA3)/(mg TA3 + TA2) in the range from 20 % to 40%. Preferably there is in step (i) made four or more of the different Teicoplanin compositions.

In a preferred embodiment the identified composition of step (iii), has a higher antibiotic activity than a corresponding Teicoplanin composition that has a %(mg TA3)/(mg TA3 + TA2) of 6.5%, where the antibiotic activity is measured by a microbiological agar diffusion assay using *Staphylococcus aureus* ATCC 6538 as test strain and the measured potential potency (IU per mg TA2 + TA3/g composition) is used to calculate the antibiotic activity.

The assay and potential potency should preferably be made as described above.

Preferably, the method for making a Teicoplanin composition comprises following further step:
(iv) making a Teicoplanin pharmaceutical composition, comprising an effective amount of Teicoplanin components or a pharmaceutically acceptable salt or derivative thereof and a pharmaceutically acceptable carrier, wherein the %(mg TA3)/(mg TA3 + TA2) of the pharmaceutical composition is a % that corresponds to the % of the identified composition of step (iii) of the third aspect of the invention.

The term "corresponds" in this step (iv) should be understood quite broadly in the sense that the specific %(mg TA3)/(mg TA3 + TA2) of the pharmaceutical composition may vary some with respect to the identified composition of step (iii) of the third aspect of the invention. The % of the identified composition may be the optimal % with respect to antibiotic activity. However, the skilled person may choose to slightly modify this in a specific Teicoplanin composition for different reasons such as e.g. production economy.

Preferably, the Teicoplanin pharmaceutical composition of step (iv) above is a Teicoplanin composition having the characteristic of a Teicoplanin composition of the first aspect and related embodiments of the present invention.

A separate aspect of the invention relates to a Teicoplanin composition as described herein that has been obtained by a method for making a Teicoplanin composition as described herein.

The skilled persons knows different routine strategies for making Teicoplanin compositions comprising with different %(mg TA3)/(mg TA3 + TA2). One way is to purify TA3 from e.g. a commercial Teicoplanin composition and use this purified TA3 to enrich a Teicoplanin composition of interest to make composition with increasing amounts of TA3. See working examples herein for an illustration of this.

TA2 may be transformed by hydrolysis into TA3, where the N-acyl-D-glucosamine group with the fatty acid tail is removed from the glycopeptide base structure (see Figure 1 herein). This hydrolysation is preferably done with the crude Teicoplanin product obtained from the fermentation. The hydrolysis can be acidic, basic or enzymatic, such as carried out by means of acid treatment (presently preferred under mild conditions) and enzymes. Another way would be to adjust the fermentation conditions in a way that will give higher TA3 amounts in the fermentation product.

Accordingly, a separate aspect of the invention relates to a method for producing a Teicoplanin composition comprising TA2 and TA3, which is enriched with TA3 (compared to starting material), said method comprising hydrolysis of a composition containing TA2, such as a composition comprising TA2 and TA3 (such as a composition (starting material) having a ratio TA3/(TA2+TA3) (w/w) of more than 0,01; 0,03; 0,05; 0,07; 0,08; 0,09; 0,10; or even more than 0,11 or 0,12), e.g. by treatment with an acid or an enzyme. The end product has preferably a ratio lower than 80%. Preferably, the hydrolysis is preformed on a crude Teicoplanin composition obtained from fermentation, without separation of the Teicoplanin components TA2 and/or TA3. The hydrolysis is preferably carried out under mild conditions, such as under conditions where pH is higher than 0, such as higher than 1, or 2, or even higher than 3. When the hydrolysis is carried out as an acidic hydrolysis, it is presently preferred that the hydrolysis is carried out at a pH value between 0 and 5, or by using hydrochloric acid at a concentration between 0,1N and 1 N. The Teicoplanin can be dissolved in a polar organic solvent (such as an alcohol) or a mixture herewith with water during the hydrolysis. An optional step of the method for producing the Teicoplanin composition comprises mixing of a composition comprising TA3 and/or TA2 with the hydrolysis product, in order to obtain a standardized Teicoplanin composition. The added composition itself may be produced by hydrolysation or by fermentation. Advantageously, the hydrolysis of TA2 into TA3 should be partly, in order to directly obtain a Teicoplanin composition having a desired ratio TA3/(TA2+TA3).

A Teicoplanin composition, obtainable by the method of the third or fourth aspect of the invention, is also an aspect of the present invention. The obtainable composition has preferably a ratio TA3/(TA2+TA3) (w/w) of less than 0,95 (such as less than 0,9; 0,8; 0,7: 0,6; 0,5; 0,4 or even less than 0,35) and preferably a ratio TA3/(TA2+TA3) (w/w) of more than 0,05 (such as more than 0,07; 0,08, 0,09; 0,10; 0,11; 0,12; 0,13; 0,14; 0,15; 0,16; 0,17; 0,18; 0,19; 0,20; 0,21; 0,22; 0,23 or even more than 0,25). In a preferred embodiment, the ratio TA3/(TA2+TA3) (w/w) in the Teicoplanin composition is higher than 5% but lower than 90%; and the ratio TA3/(TA2+TA3) (w/w) is different from 6,5% (e.g. outside the range 6,25% to 6,75%, or outside the range 6% to 7%) as well as different from 10% (e.g. outside the range 9,75% to 10,25%, or outside the range 9,5% to 10,5%). TA3 is preferably TA3-1 and/or TA2 is preferably the sum: (TA2-1 + TA2-2 + TA2-3 + TA2-4 + TA2-5). Presently, most preferred is a Teicoplanin composition of the first aspect of the invention or the embodiments thereof as described herein.

### EXAMPLES:

### Example 1: Analyzing the synergistic effect of TA3-1 and TA2-2 in different compositions.

### 1.1 MATERIALS

### 1.2 EQUIPMENT

### 1.3 METHODS

### 1.3.1 PURIFICATION OF TEICOPLANIN A3-1

### 1.3.2 PURIFICATION OF TEICOPLANIN A2-2

### 1.3.3 HPLC-ANALYSIS

### 1.3.4 DETERMINATION OF POTENCY

### 2 RESULTS

### 3 DISCUSSION

### 4 CONCLUSION

### 1.1: MATERIALS

The following Teicoplanin sources are used.

| Source | Name | Lot |
|---|---|---|
| Aventis | Targocid | 031951 and 021972 |
| Alpharma | | In house batches |
| Richet | Teicoplanina | 22188 |
| Xinchang Pharma (China) | Bulk Teicoplanin | 991022-6 |
| WHO^{*)} | International standard | WHO/NIBSC 90/704 |

| | | |
|---|---|---|
| *) For analysis a working standard is used, which has been calibrated against the WHO international standard. | | |

Unless otherwise specified, all other materials described in the examples are standard laboratory chemicals.

### 1.2 Equipment

Unless otherwise specified, all equipment described in the examples is standard laboratory relevant equipment.

### 1.3 Methods

### 1.3.1 Purification of Teicoplanin A3-1

To prepare a crude product with increased content of TA3-1 (Alpharma produced) Teicoplanin TA2 in 50% ethanol solution was converted by acid hydrolysis (1M HCl was added, resulting pH=0.6) at elevated temperatures to a crude mixture of TA2 and TA3 (including TA3-2 and the T-aglycone) and freeze dried.
To prepare pure TA3-1 Chinese material was purified by preparative reversed phase C18 HPLC. The resulting solution was polished by normal phase HPLC to remove excess salt and dried to form a powder. The identity of the components was confirmed by FIA-LC-MS.

### 1.3.2 Purification of Teicoplanin A2-2

Pure TA2-2 component was prepared by utilizing the same methods as for TA3-1.

### 1.3.3 HPLC-analysis for determination of activity

The HPLC method routinely used in the down stream process laboratory:

| | | |
|---|---|---|
| Column: | Waters Xterra^{®} RP18, 3.5 µm, 4.6 x 150 mm | |
| Precolumn: | Waters Xterra^{®} RP18 Guard column, 5 µm, 3.9 x 20 mm | |
| Column oven: | 28°C | |
| Buffer: | 0.2 % ammonium formate, pH 7.0 | |
| | 4.0 g ammonium formate was dissolved in approximately 21 of Milli-Q water. pH was adjusted to 7.0 with NH₃. The total volume was adjusted to accurately 2.0 1 with Milli-Q water. The buffer was filtered through a 0.45 µm filter before use. | |
| Mobile phase: | Eluent A: | 90 % buffer |
| | | 10 % acetonitrile (ACN) |
| | Eluent B: | 35 % buffer |
| | | 65 % ACN |

The mobile phase gradient is presented in Table 1.

**Table 1. Gradient utilized for the HPLC-analysis**

| Step | Time | Flow | % A | % B | Curve¹ |
|---|---|---|---|---|---|
| | (min) | (ml/min) | v/v | v/v | |
| 0 | 0 | 1,5 | 85 | 15 | 6 |
| 1 | 1 | 1,3 | 73 | 27 | 6 |
| 2 | 3.5 | 1,3 | 72 | 28 | 11 |
| 3 | 13 | 1,5 | 60 | 40 | 6 |
| 6 | 13.5 | 1,5 | 0 | 100 | 6 |
| 7 | 15 | 1,5 | 0 | 100 | 6 |
| 8 | 16 | 1,5 | 85 | 15 | 6 |
| 9 | 23 | 1,5 | 85 | 15 | 6 |
| 10 | 30 | 0,05 | 85 | 15 | 6 |

| | | | | | |
|---|---|---|---|---|---|
| ¹6 refers to a linear gradient and 11 refers to a delayed step gradient | | | | | |

Detection: UV, 280 nm

### Standard: Targocid, 0.210 g/l.

10 to 200 µl, corresponding to 2.1-42 µg Teicoplanin A2, was injected for a 5-point calibration curve, recorded in the beginning of the sequence and after every 30^{th} sample. Variance in the response (= the total area of the five TA2 components) up to +/- 3 % was accepted. Variance in retention time up to 10 % was also accepted.

### Preparation of downstream samples:

Liquid samples were diluted with 25 mM HEPES-buffer, pH 7, until approximately 0.25 - 2 g/l and µl of the diluted sample were injected. Solutions used for potency determinations were prepared and 20µl directly injected.
Powders were weighed, approximately 25 mg, into a 25 ml measuring flask and dissolved in 25 mM HEPES-buffer, pH 7 and 20 µl were injected.

Preparation of HEPES-buffer: 2.98 g HEPES was dissolved in approximately 450 ml of Milli-Q water. pH was adjusted to 7.0 with NaOH. The total volume was adjusted to accurately 500 ml with Milli-Q water.

The chromatograms were automatically integrated in range of 3 to 13 minutes by "valley to valley baseline" in the Waters Millenium software and the areas of the defined peaks TA3-1, TA2-1, TA2-2, TA2-3, TA2-4 and TA2-5 were recorded. All mass calculations (mg activity/g powder) were performed automatically by the software utilizing the calibration curve for TA2 and the sequence information about the weight of the samples and the dilutions. The combined area of the five TA2 components was used for calculations of the content of mg TA2/g powder. To calculate the content of TA3-1 the area of that peak was multiplied by a factor of 0.83 to compensate for the lower molecular weight of TA3-1. The percentage distribution of TA3-1 and TA2 was manually calculated by dividing the respective mass of TA3-1 and TA2 respectively by the total mass, i.e. mg(TA3-1) / mg(TA3-1 + TA2).

### 1.3.4 Determination of potency

Potency of Teicoplanin TA2, TA3-1 alone or in combinations was determined by agar diffusion assays. Furthermore, the antimicrobial effect of pure and spiked products was investigated through the determination of the minimum inhibitory concentration (MIC) by a modified "MIC" test in micro titre trays.

### 1.3.4.1 Determination by agar diffusion

### Microbiological potency by agar diffusion

The potency test is carried out according to "Microbiological assay, Teicoplanin" described below. The assay method generally corresponds to USP 25 and Ph.Eur 4^{th}. ed., but differs from both with respect to test strain and from the USP with respect to agar holes (ponds) as preferred to cylinders. The test is carried out as a 2,2 design as described in section <111> of the USP.

### Materials and specific equipment

| | |
|---|---|
| Media: | Antibiotic nutrient agar (Difco) art: 0001-01-8. |
| Buffer: | 0.2M phosphate buffer, pH 8.0 |
| Test organism: | *Staphylococcus aureus* ATCC 6538 or *Bacillus subtilis* ATCC 6633. |
| Antibiotic standard: | Working standard calibrated against the USP standard and Ph.Eur. standard. Alternatively USP or Ph.Eur. ref. standard. |
| Large plates: | Glass with aluminium frame (28,5 x 28,5 cm). |

### Procedure

Weighing and dissolution of standard and samples for stock solutions, approx. 900 IU/ml:

Teicoplanin standard: approx. 25.0 mg in 4.0 ml water and buffer ad 25.0 ml. Sample: approx. 25.0 mg in 4.0ml water and buffer ad 25.0 ml. Adjust the amount of sample according to the actual amount of standard weighed.

Dilute the standard and sample with buffer, to the following concentrations: 2-point assay:

| | |
|---|---|
| Solution 1 High: | approx. 400 IU/ml, |
| Solution 2 Low: | approx. 100 IU/ml, |

Apply to plates in accordance with an 8 x 8 Latin square design.

Immediately after application, place the plates in the incubator at 34.0°C ± 2°C, for 16-18 hours.

### Results

Read the zone diameters carefully with a 10x magnification and a precision of 0.1 mm.
Statistical evaluation: For calculation and validation, follow the guidelines set out in Ph. Eur. and USP. Use a computer program for all calculations.
In the spiking experiments the amount of the investigated sample (amount of powder weighed) was reduced and replaced by a corresponding amount of spiking material or stock solutions were mixed in defined ratios.

### 1.3.4.2 Determination of MIC-values

Pure TA3-1, (Teicoplanin R59.193.9 96% TA2-X, 4% TA3-1) and mixtures thereof were used. The test strain used was *S.aureus* ATCC 6538 in final concentrations of 5x10⁵/ml and 5x10⁴/ml respectively. The test was carried out in micro titer trays as a modification of an ordinary MIC test. Instead of using two-fold dilutions, 0,40-1,20 µg/ml with 0.20 µl divisions and 1.0-4.0 µl/ml with 1.0 µl divisions was used. The trays were incubated at 37°C. The wells were examined after 24 hours and any visible growth was noted. The "MIC" value was defined as the lowest Teicoplanin concentration, which did not show visible growth.

### 2. Results

A series of spiking experiments with different levels of TA3-1 were performed and the potency of the resulting mixtures was determined by agar diffusion. The results are summarized in Table 2.

**Table 2 Results from spiking experiments**

| Sample | TA2 content mg / g powder | TA3 content mg / g powder | Potency IU/mg powder | Potential potency IU/ mg (TA2 + TA3-1) |
|---|---|---|---|---|
| Targocid workine std | 690 | 48 | 917 | 1243 |
| TA3-1 TKO-002-03-04 | 1 | 829 | 570 | 687 |
| Richet | 612 | 32 | 811 | 1226 |
| Richet | 605 | 32 | 761 | 1195 |
| Richet + 5% TA3-1 | 627 | 67 | 856 | 1233 |
| Richet + 10% TA3-1 | 601 | 96 | 896 | 1286 |
| Richet + 15% TA3-1 | 562 | 142 | 987 | 1402 |
| Richet + 20% TA3-1 | 528 | 183 | 1054 | 1482 |
| Richet + 25% TA3-1 | 504 | 214 | 1068 | 1487 |
| R59.174.NaCl | 585 | 27 | 745 | 1217 |
| R59.174.NaC1 + 11.8% TA3-1 | | | 890 | |
| R59.174.NaCl + 13.5% TA3-1 | 581 | 156 | 980 | 1330 |
| R59.174.NaCl + 20% TA3-1 | 555 | 222 | 1020 | 1312 |
| R59.174.NaCl + 21.1% TA3-1 | | | 965 | |
| R59.193.9 | 731 | 27 | 828 | 1092 |
| R59.193.9 + 5% TA3-1 | 705 | 68 | 930 | 1204 |
| R59.195.28 | 788 | 19 | 982 | 1217 |
| R59.195.28 + 5% TA3-1 | 750 | 55 | 988 | 1227 |
| R59.195.28 + 10% TA3-1 | 721 | 96 | 1061 | 1299 |
| R59.195.28 + 15% TA3-1 | 680 | 132 | 1124 | 1384 |
| R59.195.28 + 20% TA3-1 | 632 | 177 | 1163 | 1438 |
| R59.195.28 + 25% TA3-1 | 584 | 224 | 1214 | 1502 |
| TA2-2 TKO-002-03-05 | 770 | 23 | 807 | 1017 |
| TA2-2 + 5% TA3-1 | 744 | 65 | 874 | 1080 |
| TA2-2 + 10% TA3-1 | 694 | 106 | 955 | 1194 |
| TA2-2 TKO-002-03-05 | 767 | 14 | 776 | 994 |
| TA2-2 + 5% TA3-1 | 785 | 49 | 853 | 1023 |
| TA2-2 + 10% TA3-1 | 753 | 81 | 886 | 1062 |
| TA2-2 + 15% TA3-1 | 709 | 121 | 990 | 1193 |
| TA2-2 + 20% TA3-1 | 666 | 166 | 1065 | 1280 |
| TA2-2 + 25% TA3-1 | | | 1093 | |

| | | | | |
|---|---|---|---|---|
| TA2 is the total content of TA2-1 to TA2-5, and TA3 is the content of TA3-1. | | | | |

All spiking experiments clearly revealed a synergistic effect of TA3-1 in the potency tests by agar diffusion. In order to strengthen the evidence some experiments were performed with a modified antimicrobial analysis of Teicoplanin, spiked with different amounts of TA3-1. It was carried out in liquid medium in contrast to the agar diffusion tests and determined the MIC values. The results are summarized in Table 3.

**Table 3 "MIC"-values of Teicoplanin and /or TA3-1 on S.aureus ATCC6538**

| Sample | MIC (µg/ml) | MIC (µg/ml) |
|---|---|---|
| | 5x10⁵/ml *S.aureus* | 5x10⁴/ml *S.aureus* |
| R59.193.9 (a) | 0,6 | 0,4-0,6 |
| TA3-1 (b) | 2-3 | 2-3 |
| 90% (a) + 10% (b) | 0,4-0,6 | 0,4 |

### 3. Discussion

The results from this study indicate a much lower potency of TA3-1 (570 IU/mg powder) than that of TA2-2 (807 IU/mg). Just adding the effect of the content of A3-1 in high potency products would thus not by itself explain the higher potency. The only reasonable explanation would be that TA3 adds a synergistic effect.

In this study we added different amounts of TA3 to a range of different Teicoplanin products, including the purified TA2-2 single component, with variable microbial potencies alone. Replacing from 5 to 25 % of Teicoplanin sample with the corresponding amount of TA3-1 invariably increased the microbial potency of the sample, measured in IU/mg powder. The data also proved that the potential (= the actual quality of the Teicoplanin in the powder) increased when active TA2 was replaced by low active TA3-1, indicating a synergistic effect between TA2 and TA3. The results can be visualized in a graphic plot as shown in Figure 3 herein.

Comparing the potential potencies (IU/mg TA2+TA3-1) in Fig 3, a synergistic effect of TA3-1 with the other Teicoplanin components is visible in the plot. Even products with high potency are improved when some of the TA2 is replaced with TA3-1.

As also can be seen from Figure 3 the synergistic effect seems to occur when the ratio of TA3-1 to total TA2 + TA3-1 is above 5 % and to increase steadily to at least around 25 to 30 % where it may subside slightly although further spiking was not investigated.

The results from the MIC determination supported the findings by agar diffusion assays. The antimicrobial effect of TA3-1 alone on *S. aureus* was much lower than that of Teicoplanin.

### 4. Conclusion

Through this study we have gathered evidence for the proposed synergy effect between Teicoplanin A2 and A3. The mechanism of this synergy is yet unclear, but both agar diffusion and MIC determinations of pure and spiked samples have shown there is a true synergistic effect between TA3 and TA2.

### REFERENCES:

Reference 1 *Goldstein, B. et al, (1994), Teicoplanin in Glycopeptide Antibiotics, ed. Nagarajan, R., Marcel Dekker, Inc., N.Y, N.Y., Chapter 8, pp. 273-307.*
Reference 2 Parenti, F. et al, (2000), Teicoplanin Chemistry and Microbiology, J. Chemotherapy, 12: 5-14.
Reference 3 Yao, R.C. and Crandall, L.W., (1994), Glycopeptides, Classification, Occurrence, and Discovery in Glycopeptide antibiotics, ed. Nagarajan, R., Marcel Dekker, Inc., N.Y, N.Y., Chapter 1, pp. 1-27.
Reference 4 Janknegt, R., (1991), Teicoplanin in Perspective, Pharmaceutisch Weekblad Scientific Edition, 13: 153-160.
Reference 5 Reynolds P. E., (1989), Structure, Biochemistry and Mechanism of Action of Glycopeptide Antibiotics, Review, Eur. J. Clin. Microbiol. Infect. Dis., vol. 8, No. 11: 943-950.
Reference 7 Malabarba, A. et al. (1984), Teicoplanin, Antibiotics from Actinoplanes teichomyceticus nov. sp., VI. Chemical Degradation: Physico-chemical and Biological Properties of Acid Hydrolysis Products, The Journal of Antibiotics, Vol. XXXVII, no 9, 988-999.
Reference 8 Malabarba, A. et al., (1987), Glycopeptide Antibiotic L 17054, US 4,645,827
Reference 9 US4239751
Reference 10 US4994555
Reference 11 Coronelli, C. et al: "Teicoplanin: chemical, physicochemical and biological aspects" Farmaco, Edizione Scientifica, (1987), 42(10), 767-86.

## Claims

1. A Teicoplanin composition, comprising an effective amount of Teicoplanin components or a salt thereof, wherein the composition comprises %(mg TA3)/(mg TA3 + mg TA2) in the range from 16% to 55%, measured by HPLC analysis.

2. The Teicoplanin composition of claim 1, wherein TA2 is the total of the Teicoplanin components TA2-1 to TA2-5 and wherein TA3 is the Teicoplanin component TA3-1.

3. The Teicoplanin composition of claims 1 or 2, wherein the composition comprises %(mg TA3)/(mg TA3 + TA2) in the range from 18 % to 40%, measured by HPLC analysis.

4. The Teicoplanin composition of any of claims 1 to 3, wherein the composition has a higher antibiotic activity than a corresponding Teicoplanin composition that has a %(mg TA3)/(mg TA3 + TA2) of 6.5%, where the antibiotic activity is measured by a microbiological agar diffusion assay using Staphylococcus aureus ATCC 6538 as test strain and the measured potential potency (IU per mg TA2 + TA3) is used to calculate the antibiotic activity.

5. The Teicoplanin composition of claim 4, wherein potential potency of the Teicoplanin composition is at least 30 IU higher than the corresponding Teicoplanin composition that has a %(mg TA3)/(mg TA3 + TA2) of 6.5%.

6. Use of a Teicoplanin composition of any of the preceding claims for the manufacture of a medicament for the treatment of a microorganism infection in a patient, such as an infection with Gram-positive bacteria, in particular with staphylococcal bacteria.

7. Method for producing a Teicoplanin composition comprising TA2 and TA3, said method comprises hydrolysis of a composition containing TA2, e.g. by treatment with an acid or an enzyme, wherein the Teicoplanin composition has a ratio TA3-1/(TA2+TA3-1) (w/w) in the range from 16% to 55%.

8. The method of claim 7, wherein the hydrolysis is performed on a crude Teicoplanin composition obtained from fermentation, without separation of the Teicoplanin components TA2 and TA3.

## Patentansprüche

1. Eine Teicoplaninzusammensetzung umfassend eine wirksame Menge von Teicoplaninkomponenten oder eines Salzes davon, wobei die Zusammensetzung %(mg TA3)/(mg TA3 + mg TA2) im Bereich von 16% bis 55% umfasst, gemessen durch HPLC-Analyse.

2. Die Teicoplaninzusammensetzung nach Anspruch 1, wobei TA2 die Gesamtheit der Teicoplaninverbindungen TA2-1 bis TA2-5 ist und wobei TA3 die Teicoplaninverbindung TA3-1 ist.

3. Die Teicoplaninzusammensetzung nach Anspruch 1 oder 2, wobei die Zusammensetzung %(mg TA3)/(mg TA3 + TA2) im Bereich von 18% bis 40% umfasst, gemessen durch HPLC-Analyse.

4. Die Teicoplaninzusammensetzung nach einem der Ansprüche 1 bis 3, wobei die Zusammensetzung eine höhere antibiotische Aktivität hat als eine entsprechende Teicoplaninzusammensetzung, die ein %(mg TA3)/(mg TA3 + TA2) von 6,5% aufweist und wobei die antibiotische Aktivität durch einen mikrobiologischen Agar-Diffusionsassay unter Verwendung von *Staphylococcus aureus* ATCC 6538 als Teststamm gemessen wird und die gemessene potentielle Wirksamkeit (IE pro mg TA2 + TA3) verwendet wird, um die antibiotische Aktivität zu berechnen.

5. Die Teicoplaninzusammensetzung nach Anspruch 4, wobei die potentielle Wirksamkeit der Teicoplaninzusammensetzung um mindestens 30 IE höher ist als die der entsprechenden Teicoplaninzusammensetzung, die ein %(mg TA3)/(mg TA3 + TA2) von 6,5% aufweist.

6. Verwendung einer Teicoplaninzusammensetzung nach einem der vorhergehenden Ansprüche für die Herstellung eines Medikaments für die Behandlung einer Infektion mit Mikroorganismen in einem Patienten, wie eine Infektion mit Gram-positiven Bakterien, insbesondere mit Staphylokokken.

7. Verfahren zur Herstellung einer Teicoplaninzusammensetzung umfassend TA2 und TA3, das Verfahren umfassend Hydrolyse einer TA2-haltigen Zusammensetzung, z. B. durch Behandlung mit einer Säure oder einem Enzym, wobei die Teicoplaninzusammensetzung ein Verhältnis von TA3-1/(TA2+TA3-1) (Gewicht/Gewicht) im Bereich von 16% bis 55% aufweist.

8. Verfahren nach Anspruch 7, wobei die Hydrolyse mit einer unbehandelten, durch Fermentation erhaltenen, Teicoplaninverbindung, ohne Abtrennung der Teicoplaninverbindungen TA2 und TA3, durchgeführt wurde.

## Revendications

1. Composition de téicoplanine, comprenant une quantité efficace de composants de téicoplanine ou d'un sel de ceux-ci, la composition ayant un pourcentage (mg de TA3)/(mg de TA3 + mg de TA2) dans la plage de 16 % à 55 %, mesuré par analyse HPLC.

2. La composition de téicoplanine de la revendication 1, dans laquelle TA2 représente la totalité des composants de téicoplanine TA2-1 à TA2-5 et dans laquelle TA3 représente le composant de téicoplanine TA3-1.

3. La composition de téicoplanine de la revendication 1 ou 2, la composition ayant un pourcentage (mg de TA3)/(mg de TA3 + TA2) dans la plage de 18 % à 40 %, mesuré par analyse HPLC.

4. La composition de téicoplanine selon l'une quelconque des revendications 1 à 3, la composition ayant une activité antibiotique plus élevée qu'une composition de téicoplanine correspondante ayant un pourcentage (mg de TA3)/(mg de TA3 + TA2) de 6,5 %, l'activité antibiotique étant mesurée par un essai microbiologique de diffusion en milieu gélosé en employant Staphylococcus aureus ATCC 6538 en tant que souche d'essai, et la puissance potentielle mesurée (UI par mg de TA2 + TA3) est employée pour calculer l'activité antibiotique.

5. La composition de téicoplanine de la revendication 4, la puissance potentielle de la composition de téicoplanine étant d'au moins 30 UI plus élevée que la composition de téicoplanine correspondante ayant un pourcentage (mg de TA3)/(mg de TA3 + TA2) de 6,5 %.

6. Utilisation d'une composition de téicoplanine de l'une quelconque des revendications précédentes, pour la fabrication d'un médicament destiné au traitement d'une infection par un microorganisme chez un patient, telle qu'une infection par une bactérie Gram-positive, en particulier par une bactérie de type staphylocoque.

7. Procédé pour la production d'une composition de téicoplanine, comprenant TA2 et TA3, ledit procédé comprenant l'hydrolyse d'une composition contenant TA2, par exemple par traitement avec un acide ou une enzyme, et la composition de téicoplanine ayant un rapport TA3-1/(TA2 + TA3-1) en poids dans la plage de 16 % à 55 %.

8. Le procédé de la revendication 7, dans lequel l'hydrolyse est effectuée sur une composition de téicoplanine brute obtenue par fermentation, sans séparation des composants TA2 et TA3.
